# DEMANDE DE BREVET EUROPEEN

(11) **EP 1 559 407 A1**
(43) Date de publication de la demande: **03.08.2005**
(21) Numéro de dépôt: 05290085.9
(22) Date de dépôt: 14.01.2005
(51) Int. Cl.: A61K 7/13

(54) **Composition de teinture des fibres kératiniques contenant une alcool oxydase et un polymère associatif de type polyuréthane, procédé mettant en oeuvre cette composition**

(30) Priorité: 28.01.2004 FR 0400779
(71) Demandeur: L'OREAL, 75008 Paris (FR)
(72) Inventeur: Plos, Grégory, Tokyo 158-0097 (JP)
(74) Mandataire: Casalonga, Axel

(57) **Abrégé**

La présente demande concerne une composition pour la teinture des fibres kératiniques, et en particulier les fibres kératiniques humaines telles que les cheveux, contenant, dans un milieu approprié pour la teinture, au moins un précurseur de colorant d'oxydation, au moins une enzyme alcool oxydase, au moins un substrat pour ladite enzyme et au moins un polymère associatif de type polyuréthane. Cette demande concerne encore un procédé de teinture des fibres kératiniques qui consiste à appliquer cette composition ainsi qu'un "kit" de teinture.

## Description

La présente invention a pour objet une composition pour la teinture des fibres kératiniques, en particulier des fibres kératiniques humaines telles que les cheveux, contenant, dans un milieu approprié pour la teinture, au moins un précurseur de colorant d'oxydation, au moins une enzyme alcool oxydase, au moins un substrat pour ladite enzyme et au moins un polymère associatif de type polyuréthane.

Il est connu de teindre les fibres kératiniques et en particulier les cheveux humains avec des compositions tinctoriales contenant des précurseurs de colorant d'oxydation, appelés généralement bases d'oxydation, tels que des ortho ou paraphénylènediamines, des ortho ou paraaminophénols et des composés hétérocycliques. Ces bases d'oxydation sont des composés incolores ou faiblement colorés qui, associés à des produits oxydants, peuvent donner naissance à des composés colorés par un processus de condensation oxydative.

On sait également que l'on peut faire varier les nuances obtenues avec ces bases d'oxydation en les associant à des coupleurs ou modificateurs de coloration, ces derniers étant choisis notamment parmi les métadiamines aromatiques, les métaaminophénols, les métadiphénols et certains composés hétérocycliques tels que des composés indoliques.

La variété des molécules mises en jeu au niveau des bases d'oxydation et des coupleurs permet l'obtention d'une riche palette de couleurs.

La coloration dite "permanente" obtenue grâce à ces colorants d'oxydation, doit par ailleurs satisfaire un certain nombre d'exigences. Ainsi, elle doit être sans inconvénient sur le plan toxicologique, elle doit permettre d'obtenir des nuances dans l'intensité souhaitée et présenter une bonne tenue face aux agents extérieurs tels que la lumière, les intempéries, le lavage, les ondulations permanentes, la transpiration et les frottements.

Les colorants doivent également permettre de couvrir les cheveux blancs, et être enfin les moins sélectifs possibles, c'est-à-dire permettre d'obtenir des écarts de coloration les plus faibles possibles tout au long d'une même fibre kératinique, qui est en général différemment sensibilisée (c'est-à-dire abîmée) entre sa pointe et sa racine.

La coloration est généralement réalisée en milieu fortement alcalin, en présence de peroxyde d'hydrogène. Toutefois, l'utilisation des milieux alcalins en présence de peroxyde d'hydrogène présente pour inconvénient d'entraîner une dégradation non négligeable des fibres, ainsi qu'une décoloration des fibres kératiniques qui n'est pas toujours souhaitable.

La coloration d'oxydation des fibres kératiniques peut également être réalisée à l'aide de systèmes oxydants différents du peroxyde d'hydrogène tels que des systèmes enzymatiques. Ainsi la demande de brevet FR 2 769 219 décrit l'utilisation d'une enzyme uricase et de son substrat l'acide urique en coloration d'oxydation pour la teinture de fibres kératiniques. La demande EP-A-0 310 675 décrit l'utilisation de précurseur de colorant d'oxydation de type benzénique en association avec des enzymes telles que la pyranose-oxydase, la glucose-oxydase. Plus récemment, la demande de brevet FR 2 833 492 décrit l'utilisation de l'enzyme alcool-oxydase en tant que seule enzyme dans une composition de coloration d'oxydation pour la teinture de fibres kératiniques.

Le but de la présente invention est de fournir de nouvelles compositions pour la teinture des fibres kératiniques par coloration d'oxydation épaissies et stables, utilisant un système oxydant différent du peroxyde d'hydrogène.

Par " composition épaissie", au sens de la présente demande on entend une composition qui peut être appliquée sur la zone à teindre sans couler hors de cette zone.

De préférence, la viscosité des compositions selon l'invention est supérieure à 200 cp de manière encore plus préférée supérieure à 500 cp, mesurée à 25°C à l'aide d'un rhéomètre RHEOMAT RM 180 à un taux de cisaillement de 200 s⁻¹.

De manière avantageuse et inattendue, la demanderesse vient maintenant de découvrir qu'il est possible d'atteindre ce but en utilisant au moins un précurseur de colorant d'oxydation, au moins une enzyme alcool oxydase, au moins un substrat pour ladite enzyme et au moins un polymère associatif de type polyuréthane dans une composition pour la teinture des fibres kératiniques, en particulier des fibres kératiniques humaines telles que les cheveux.

La composition selon l'invention permet l'obtention de formulations particulièrement homogènes qui, une fois appliquées, respectent la nature des fibres kératiniques et ne présentent pas les problèmes de solubilisation et de cristallisation rencontrés notamment avec le système acide urique/ uricase. La demanderesse a aussi noté que la stabilité de la composition selon l'invention et en particulier la stabilité de l'enzyme alcool oxydase de cette composition est améliorée.

Les compositions selon la présente invention présentent l'avantage de conduire à l'obtention de teintures de couleurs puissantes, peu sélectives et résistantes, ces compositions sont capables d'engendrer des nuances variées de couleur intense et uniforme, sans dégradation significative du cheveu. En outre, on a noté que l'utilisation d'une telle composition améliore la tenue des cheveux permanentés et diminue la porosité du cheveu.

La Demanderesse a également observé que, lors de l'utilisation de la composition selon l'invention, la montée du colorant sur les fibres n'est pas freinée, ce qui est généralement le cas lors de l'emploi de compositions de coloration contenant des épaississants traditionnels, tensio-actifs et solvants.

Or, lorsque la montée du colorant sur les fibres est freinée, l'obtention d'une nuance puissante nécessite alors l'utilisation d'une quantité plus importante de colorant et également d'une quantité plus importante de solvant et/ou de tensio-actif pour solubiliser ce colorant.

D'autres caractéristiques, aspects, objets et avantages de la présente invention apparaîtront encore plus clairement à la lecture de la description et des exemples qui suivent.

Les polymères associatifs sont des polymères de préférence hydrosolubles capables, en milieu aqueux, de s'associer réversiblement entre eux ou avec d'autres molécules pour conduire à un épaississement accru du milieu.

Les polymères associatifs de type polyuréthanes utilisables dans les compositions selon la présente invention possèdent au moins une chaîne grasse terminale ou pendante comportant au moins dix atomes de carbone. Ces polymères sont susceptibles d'interagir avec eux-mêmes ou avec des composés particuliers du milieu dans lequel ils se trouvent tels que des tensio-actifs pour conduire à un épaississement du milieu. Les polymères associatifs de type polyuréthanes peuvent être cationiques, anioniques ou non ioniques.

À titre de polymères associatifs de type polyuréthanes cationiques, on peut utiliser les composés dont la famille a été décrite par la Demanderesse dans la demande de brevet français N° 0009609; elle peut être représentée par la formule générale (I) suivante :

R-X-(P)n-[L-(Y)m]r-L'-(P')p-X'-R' (I)

dans laquelle :
R et R', identiques ou différents, représentent un groupement hydrophobe ou un atome d'hydrogène ;
X et X', identiques ou différents, représentent un groupement comportant une fonction amine portant ou non un groupement hydrophobe, ou encore le groupement L" ;
L, L' et L", identiques ou différents, représentent un groupement dérivé d'un diisocyanate ;
P et P', identiques ou différents, représentent un groupement comportant une fonction amine portant ou non un groupement hydrophobe ;
Y représente un groupement hydrophile ;
r est un nombre entier compris entre 1 et 100, de préférence entre 1 et 50 et en particulier entre 1 et 25,
n, m, et p valent chacun indépendamment des autres entre 0 et 1000 ;
la molécule contenant au moins une fonction amine quaternaire (amine protonée) ou quaternisée et au moins un groupement hydrophobe.

Dans un mode de réalisation préféré de ces polyuréthanes, les seuls groupements hydrophobes sont les groupes R et R' aux extrémités de chaîne.

Une famille préférée de polyuréthanes associatifs cationiques est celle correspondant à la formule (I) décrite ci-dessus et dans laquelle :
R et R' représentent tous les deux indépendamment un groupement hydrophobe, X, X' représentent chacun un groupe L'', n et p valent entre 1 et 1000 et L, L', L'', P, P', Y et m ont la signification indiquée ci-dessus.

Une famille préférée de polyuréthanes associatifs cationiques est celle correspondant à la formule (I) ci-dessus dans laquelle :
R et R' représentent tous les deux indépendamment un groupement hydrophobe, X, X' représentent chacun un groupe L", n et p valent 0, et L, L', L", Y et m ont la signification indiquée ci-dessus.

Le fait que n et p valent 0 signifie que ces polymères ne comportent pas de motifs dérivés d'un monomère à fonction amine, incorporé dans le polymère lors de la polycondensation. Les fonctions amines protonées de ces polyuréthanes résultent de l'hydrolyse de fonctions isocyanates, en excès, en bout de chaîne, suivie de l'alkylation des fonctions amines primaires formées par des agents d'alkylation à groupe hydrophobe, c'est-à-dire des composés de type RQ ou R'Q, dans lequel R et R' sont tels que définis plus haut et Q désigne un groupe partant tel qu'un halogénure, un sulfate etc.

Une autre famille préférée de polyuréthanes associatifs cationiques est celle correspondant à la formule (I) ci-dessus dans laquelle :
R et R' représentent tous les deux indépendamment un groupement hydrophobe, X et X' représentent tous les deux indépendamment un groupement comportant une amine quaternaire, n et p valent zéro, et L, L', Y et m ont la signification indiquée ci-dessus.

La masse moléculaire moyenne en nombre des polyuréthanes associatifs cationiques est comprise de préférence entre 400 et 500 000, en particulier entre 1000 et 400 000 et idéalement entre 1000 et 300 000.

Par groupement hydrophobe, on entend un radical ou polymère à chaîne hydrocarbonée, saturée ou non, linéaire ou ramifiée, pouvant contenir un ou plusieurs hétéroatomes tels que P, O, N, S, ou un radical à chaîne perfluorée ou siliconée. Lorsqu'il désigne un radical hydrocarboné, le groupement hydrophobe comporte au moins 10 atomes de carbone, de préférence de 10 à 30 atomes de carbone, en particulier de 12 à 30 atomes de carbone et plus préférentiellement de 18 à 30 atomes de carbone.

Préférentiellement, le groupement hydrocarboné provient d'un composé monofonctionnel.

A titre d'exemple, le groupement hydrophobe peut être issu d'un alcool gras tel que l'alcool stéarylique, l'alcool dodécylique, l'alcool décylique. Il peut également désigner un polymère hydrocarboné tel que par exemple le polybutadiène.

Lorsque X et/ou X' désignent un groupement comportant une amine tertiaire ou quaternaire, X et/ou X' peuvent représenter l'une des formules suivantes : 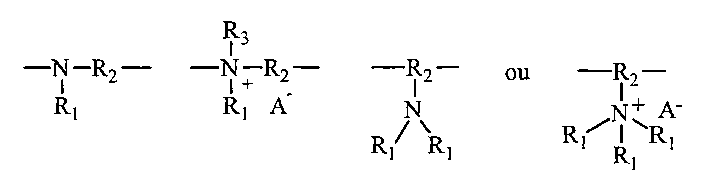 pour X 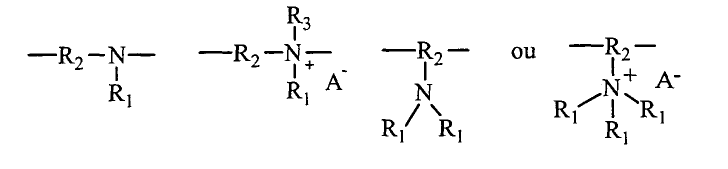 pour X'
dans lesquelles :
R₂ représente un radical alkylène ayant de 1 à 20 atomes de carbone, linéaire ou ramifié, comportant ou non un cycle saturé ou insaturé, ou un radical arylène, un ou plusieurs des atomes de carbone pouvant être remplacé par un hétéroatome choisi parmi N, S, O, P ;
R₁ et R₃, identiques ou différents, désignent un radical alkyle ou alcényle en C₁-C₃₀, linéaire ou ramifié, un radical aryle, pouvant contenir un ou plusieurs hétéroatomes (1,2,3) choisis parmi N, S, O, P ; A⁻ est un contre-ion physiologiquement acceptable.

Les groupements L, L' et L" représentent un groupe de formule : 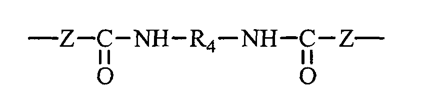 dans laquelle :
Z représente ―O-, -S- ou ―NH- ; et
R₄ représente un radical alkylène ayant de 1 à 20 atomes de carbone, linéaire ou ramifié, comportant ou non un cycle saturé ou insaturé, ou un radical arylène, pouvant contenir un ou plusieurs hétéroatomes (1,2,3) de préférence choisis parmi N, S, O et P.

Les groupements P et P', comprenant une fonction amine peuvent représenter au moins l'une des formules suivantes : 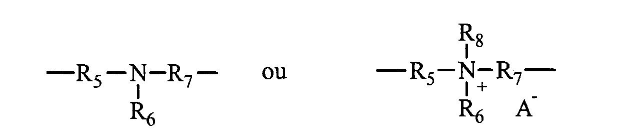 ou 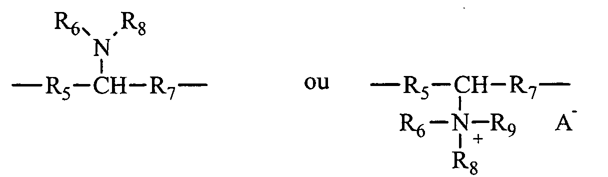 ou 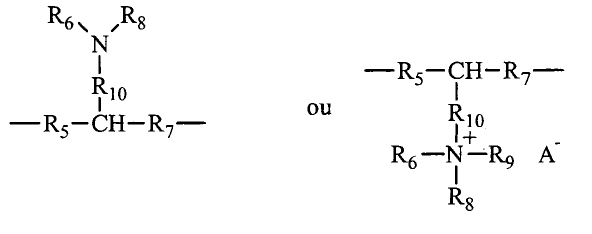 dans lesquelles :
R₅ et R₇ ont les mêmes significations que R₂ défini précédemment; R₆, R₈ et R₉ ont les mêmes significations que R₁ et R₃ définis précédemment ; R₁₀ représente un groupe alkylène de préférence en C₁-C₃₀, de manière encore préférée en C₁-C₂₀ et plus particulièrement en C₁-C₁₀, linéaire ou ramifié, éventuellement insaturé et pouvant contenir un ou plusieurs hétéroatomes (1,2,3) choisis parmi N, O, S et P, et A⁻ est un contre-ion physiologiquement acceptable.

En ce qui concerne la signification de Y, on entend par groupement hydrophile, un groupement hydrosoluble polymérique ou non.

A titre d'exemple, on peut citer, lorsqu'il ne s'agit pas de polymères, l'éthylèneglycol, le diéthylèneglycol et le propylèneglycol.

Lorsqu'il s'agit, conformément à un mode de réalisation préféré, d'un polymère hydrophile, on peut citer à titre d'exemple les polyéthers, les polyesters sulfonés, les polyamides sulfonés, ou un mélange de ces polymères. A titre préférentiel, le composé hydrophile est un polyéther et notamment un poly(oxyde d'éthylène) ou poly(oxyde de propylène).

Les polyuréthanes associatifs cationiques de formule (I) utilisables selon l'invention sont formés à partir de diisocyanates et de différents composés possédant des fonctions à hydrogène labile. Les fonctions à hydrogène labile peuvent être des fonctions alcool, amine primaire ou secondaire ou thiol donnant, après réaction avec les fonctions diisocyanate, respectivement des polyuréthanes, des polyurées et des polythiourées. Le terme "polyuréthanes" utilisable selon la présente invention englobe ces trois types de polymères à savoir les polyuréthanes proprement dits, les polyurées et les polythiourées ainsi que des copolymères de ceux-ci.

Un premier type de composés entrant dans la préparation du polyuréthane de formule (I) est un composé comportant au moins un motif à fonction amine. Ce composé peut être multifonctionnel, mais préférentiellement le composé est difonctionnel, c'est-à-dire que selon un mode de réalisation préférentiel, ce composé comporte deux atomes d'hydrogène labile portés par exemple par une fonction hydroxyle, amine primaire, amine secondaire ou thiol. On peut également utiliser un mélange de composés multifonctionnels et difonctionnels dans lequel le pourcentage de composés multifonctionnels est faible.

Comme indiqué précédemment, ce composé peut comporter plus d'un motif à fonction amine. Il s'agit alors d'un polymère portant une répétition du motif à fonction amine.

Ce type de composés peut être représenté par l'une des formules suivantes :

HZ-(P)n-ZH,

ou

HZ-(P')p-ZH

dans lesquelles Z, P, P', n et p sont tels que définis plus haut.

A titre d'exemple de composés à fonction amine, on peut citer la N-méthyldiéthanolamine, la N-tert-butyl-diéthanolamine, la N-sulfoéthyldiéthanolamine.

Le deuxième composé entrant dans la préparation du polyuréthane de formule (I) est un diisocyanate correspondant à la formule :

O=C=N-R₄-N=C=O

dans laquelle R₄ est défini plus haut.

A titre d'exemple, on peut citer le méthylènediphényl-diisocyanate, le méthylènecyclohexanediisocyanate, l'isophorone-diisocyanate, le toluènediisocyanate, le naphtalènediisocyanate, le butanediisocyanate, l'hexanediisocyanate.

Un troisième composé entrant dans la préparation du polyuréthane de formule (I) est un composé hydrophobe destiné à former les groupes hydrophobes terminaux du polymère de formule (I).

Ce composé est constitué d'un groupe hydrophobe et d'une fonction à hydrogène labile, par exemple une fonction hydroxyle, amine primaire ou secondaire, ou thiol.

A titre d'exemple, ce composé peut être un alcool gras, tel que notamment l'alcool stéarylique, l'alcool dodécylique, l'alcool décylique. Lorsque ce composé comporte une chaîne polymérique, il peut s'agir par exemple du polybutadiène hydrogéné alpha-hydroxyle.

Le groupe hydrophobe du polyuréthane de formule (I) peut également résulter de la réaction de quaternisation de l'amine tertiaire du composé comportant au moins un motif amine tertiaire. Ainsi, le groupement hydrophobe est introduit par l'agent quaternisant. Cet agent quaternisant est un composé de type RQ ou R'Q, dans lequel R et R' sont tels que définis plus haut et Q désigne un groupe partant tel qu'un halogénure, un sulfate etc.

Le polyuréthane associatif cationique peut en outre comprendre une séquence hydrophile. Cette séquence est apportée par un quatrième type de composé entrant dans la préparation du polymère. Ce composé peut être multifonctionnel. Il est de préférence difonctionnel. On peut également avoir un mélange où le pourcentage en composé multifonctionnel est faible.

Les fonctions à hydrogène labile sont des fonctions alcool, amine primaire ou secondaire, ou thiol. Ce composé peut être un polymère terminé aux extrémités des chaînes par l'une de ces fonctions à hydrogène labile.

A titre d'exemple, on peut citer, lorsqu'il ne s'agit pas de polymères, l'éthylèneglycol, le diéthylèneglycol et le propylèneglycol.

Lorsqu'il s'agit d'un polymère hydrophile, on peut citer à titre d'exemple les polyéthers, les polyesters sulfonés, les polyamides sulfonés, ou un mélange de ces polymères. A titre préférentiel, le composé hydrophile est un polyéther et notamment un poly(oxyde d'éthylène) ou poly(oxyde de propylène).

Le groupe hydrophile noté Y dans la formule (I) est facultatif. En effet, les motifs à fonction amine quaternaire ou protonée peuvent suffire à apporter la solubilité ou l'hydrodispersibilité nécessaire pour ce type de polymère dans une solution aqueuse.

Bien que la présence d'un groupe Y hydrophile soit facultative, on préfère cependant des polyuréthanes associatifs cationiques comportant un tel groupe.

A titre de polyuréthane associatif anionique, on peut notamment citer un terpolymère acrylique soluble ou gonflable dans les alcalis. Il est caractérisé par le fait qu'il comprend :
a) environ 20 à 70% en poids, de préférence 25 à 55% en poids, d'un acide carboxylique à insaturation α, β-monoéthylénique ;
b) environ 20 à 80% en poids, de préférence 30 à 65% en poids, d'un monomère à insaturation monoéthylénique non tensioactif différent de a) et
c) environ 0,5 à 60% en poids, de préférence 10 à 50% en poids, d'un monomère uréthane non-ionique qui est le produit de réaction d'un tensioactif non-ionique monohydrique avec un monoisocyanate à insaturation monoéthylénique.

L'acide carboxylique à insaturation α, β-monoéthylénique a) peut être choisi parmi de nombreux acides et en particulier l'acide acrylique, l'acide méthacrylique, l'acide itaconique et l'acide maléique. L'acide méthacrylique est préféré. Une large proportion d'acide est essentielle pour donner une structure polymère qui se solubilise et donne un épaississant par réaction avec un composé alcalin comme l'hydroxyde de sodium, les alcanolamines, l'aminométhylpropanol ou l'aminométhylpropanediol.

Le terpolymère doit aussi contenir une proportion importante indiquée ci-dessus d'un monomère b) à insaturation monoéthylénique qui n'a pas de propriété tensioactive. Les monomères préférés sont ceux qui donnent des polymères insolubles dans l'eau lorsqu'ils sont homopolymérisés et sont illustrés par les acrylates et méthacrylates d'alkyle en C₁-C₄ comme l'acrylate de méthyle, l'acrylate d'éthyle, l'acrylate de butyle ou les méthacrylates correspondants. Les monomères plus particulièrement préférés sont les (méth)acrylates de méthyle et d'éthyle. D'autres monomères pouvant être utilisés sont le styrène, le vinyltoluène, l'acétate de vinyle, l'acrylonitrile et le chlorure de vinylidène. Les monomères non réactifs sont préférés, ces monomères étant ceux dans lesquels le groupe éthylénique unique est le seul groupe réactif dans les conditions de la polymérisation. Cependant, des monomères qui contiennent des groupes réactifs sous l'action de la chaleur peuvent être utilisés dans certaines situations, comme l'acrylate d'hydroxyéthyle.

Les tensioactifs non-ioniques monohydriques utilisés pour obtenir le monomère uréthane non-ionique c) sont bien connus et sont généralement des composés hydrophobes alcoxylés contenant un oxyde d'alkylène formant la partie hydrophile de la molécule. Les composés hydrophobes sont généralement constitués par un alcool aliphatique ou un alkylphénol dans lesquels une chaîne carbonée contenant au moins six atomes de carbone constitue la partie hydrophobe du tensioactif.

Les tensioactifs non-ioniques monohydriques préférés ont pour formule : 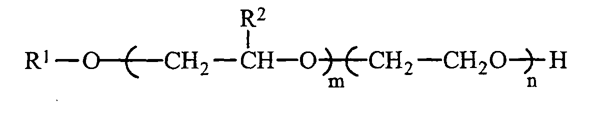 dans laquelle R¹ est un groupe alkyle en C₆-C₃₀ ou aralkyle en C₈-C₃₀, R² est un groupe alkyle en C₁-C₄, n est un nombre moyen allant d'environ 5 à 150 et m est un nombre moyen allant d'environ 0 à 50, à la condition que n soit au moins aussi grand que m et que la somme n + m soit comprise entre 5 et 150.

A titre de groupes alkyle en C₆-C₃₀ préférés, on peut citer les radicaux dodécyle et alkyle en C₁₈-C₂₆. A titre de groupes aralkyle, on peut citer plus particulièrement les groupes alkyl (C₈-C₁₃) phényle. Le groupe R² préféré est le groupe méthyle.

Le monoisocyanate à insaturation monoéthylénique utilisé pour former le monomère uréthane non-ionique c) peut être choisi parmi des composés très variés. On peut utiliser un composé contenant toute insaturation copolymérisable telle qu'une insaturation acrylique ou méthacrylique. On peut aussi utiliser une insaturation allylique conférée par l'alcool allylique. Les monoisocyanates monoéthyléniques préférés sont l'αα-diméthyl-m-isopropényl-benzylisocyanate et le méthylstyrène-isopropylisocyanate.

Le terpolymère acrylique défini ci-dessus est obtenu par copolymérisation en émulsion aqueuse des composants a), b) et c) qui est usuelle et décrite dans la demande de brevet EP-A-0 173 109.

Comme polyuréthane associatif anionique pouvant être utilisés selon la présente invention, on peut notamment citer les copolymères d'acide méthacrylique ou acrylique comprenant au moins un motif de (méth)acrylate d'alkyle en C₁₋₃₀ et un motif uréthane substitué par une chaîne grasse. On peut en particulier citer le copolymère acide méthacrylique/méthacrylate de méthyle/ méthylstyrène-isopropylisocyanate/alcool béhénylique polyéthoxylé (comportant 40 motifs éthoxy) vendu sous la marque VISCOPHOBE® DB 1000 par la société Union Carbide.

Les polyuréthanes associatifs non ioniques utilisés dans la présente invention sont notamment des polyuréthanes-polyéthers comportant dans leur chaîne, à la fois des séquences hydrophiles de nature le plus souvent polyoxyéthylénée et des séquences hydrophobes qui peuvent être des enchaînements aliphatiques seuls et/ou des enchaînements cycloaliphatiques et/ou aromatiques.

De préférence, les polyuréthane-polyéthers comportent au moins deux chaînes lipophiles hydrocarbonées, ayant de 6 à 30 atomes de carbone, séparées par une séquence hydrophile, les chaînes hydrocarbonées pouvant être des chaînes pendantes ou des chaînes en bout de séquence hydrophile. En particulier, il est possible qu'une ou plusieurs chaînes pendantes soient prévues. En outre, le polymère peut comporter, une chaîne hydrocarbonée à un bout ou aux deux bouts d'une séquence hydrophile.

Les polyuréthane-polyéthers peuvent être multiséquencés en particulier sous forme de tribloc. Les séquences hydrophobes peuvent être à chaque extrémité de la chaîne (par exemple : copolymère tribloc à séquence centrale hydrophile) ou réparties à la fois aux extrémités et dans la chaîne (copolymère multiséquencé par exemple). Ces mêmes polymères peuvent être également en greffons ou en étoile.

Les polyuréthane-polyéthers non-ioniques à chaîne grasse peuvent être des copolymères triblocs dont la séquence hydrophile est une chaîne polyoxyéthoxylée comportant de 50 à 1000 groupements éthoxylés. Les polyuréthane-polyéthers non-ioniques comportent une liaison uréthanne entre les séquences hydrophiles, d'où l'origine du nom.

Par extension figurent aussi parmi les polyuréthane-polyéthers non-ioniques à chaîne grasse, ceux dont les séquences hydrophiles sont liées aux séquences lipophiles par d'autres liaisons chimiques.

A titre de polyuréthane-polyéthers non-ioniques à chaîne grasse utilisables dans l'invention, on peut mentionner aussi le Rhéolate 205 à fonction urée vendu par la société RHEOX ou encore les Rhéolates 208, 204 ou 212,ainsi que l'Acrysol® RM 184.

On peut également citer le produit ELFACOS T210 à chaîne alkyle en C₁₂₋₁₄ et le produit ELFACOS® T212 à chaîne alkyle en C₁₈ de chez AKZO.

Le produit DW 1206B de chez ROHM & HAAS à chaîne alkyle en C₂₀ et à liaison uréthanne, proposé à 20 % en matière sèche dans l'eau, peut aussi être utilisé.

On peut aussi utiliser des solutions ou dispersions de ces polymères notamment dans l'eau ou en milieu hydroalcoolique. A titre d'exemple de tels polymères on peut citer, le Rhéolate® 255, le Rhéolate® 278 et le Rhéolate® 244 vendus par la société RHEOX. On peut aussi utiliser le produit DW 1206F et le DW 1206J proposés par la société ROHM & HAAS.

Les polyuréthane-polyéthers utilisables selon l'invention sont en particulier ceux décrits dans l'article de G. Fonnum, J. Bakke et Fk. Hansen - Colloid Polym. Sci 271, 380.389 (1993).

Comme polyuréthanes associatifs non ioniques préférés, on peut citer les polyuréthane-polyéthers susceptibles d'être obtenus par polycondensation d'au moins trois composés comprenant (i) au moins un polyéthylèneglycol comprenant de 150 à 180 moles d'oxyde d'éthylène, (ii) de l'alcool stéarylique ou de l'alcool décylique et (iii) au moins un diisocyanate.

De tels polyuréthane-polyéthers sont vendus notamment par la société ROHM & HAAS sous les appellations ACULYN ® 46 et ACULYN® 44. L'ACULYN® 46 est un polycondensat de polyéthylèneglycol à 150 ou 180 moles d'oxyde d'éthylène, d'alcool stéarylique et de méthylène-bis(4-cyclohexylisocyanate) (SMDI), à 15% en poids dans une matrice de maltodextrine (4%) et d'eau (81%); l'ACULYN® 44 est un polycondensat de polyéthylèneglycol à 150 ou 180 moles d'oxyde d'éthylène, d'alcool décylique et de méthylène-bis(4-cyclohexylisocyanate) (SMDI), à 35% en poids dans un mélange de propylèneglycol (39%) et d'eau (26%).

Les polymères associatifs de type polyuréthanes sont utilisés dans les compositions selon l'invention de préférence en une quantité comprise entre 0,01 et 10% en poids par rapport au poids total de la composition de teinture. Plus préférentiellement, cette quantité est comprise entre 0,1 et 5% en poids.

Dans le cadre de la présente invention, les enzymes alcool oxydases utilisées dans la composition tinctoriale conforme à l'invention appartiennent à la classe E.C.1.1.3 de la nomenclature des enzymes, (voir Enzyme Nomenclature, Academic Press Inc ; 1992).

Lesdites enzymes sont choisies parmi les alcool primaire oxydases ( EC1.1.3.13), les alcool secondaire oxydases (EC 1.1.3.18), les alcool à longue chaîne hydrocarbonée oxydases (EC 1.1.3.20), les alcool polyvinylique oxydases ( EC 1.1.3.30), l'alcool vanillique oxydase (EC 1.1.3.38), les alcool aromatique oxydases (EC 1.1.3.7) encore appelées aryl alcool oxydases.

De préférence l'enzyme utilisée dans la composition selon l'invention est une alcool primaire oxydase ( EC1.1.3.13).

Les enzymes alcool oxydases forment une classe particulière d'enzymes oxydo-réductase à 2 électrons.

L'enzyme alcool oxydase utilisée dans la composition tinctoriale selon l'invention peut être issue d'un extrait de végétaux, d'animaux, de microorganismes (bactérie, champignon, levure, microalgue ou virus), de cellules différenciées ou dédifférenciées, obtenues *in vivo* ou *in vitro,* modifiées ou non modifiées génétiquement, ou synthétiques (obtenues par synthèse chimique ou biotechnologique).

A titre d'exemples, l'enzyme alcool oxydase peut être issue d'une des espèces suivantes : *Rhodococcus erythropolis, Pseudomonas pseudoalcaligenes* qui sont des bactéries, *Aspergillus niger, Kamagataella pastoris, Phanerochaete chrysosporium, Polyporus obtusus, Hansenula polymorpha, Poria contigua, Penicillium simplicissimum, Pleurotus pulmonarius* (champignons), *Pichia sp. (pastoris, methanolica, angusta) et Candida sp. (boidinii, albicans, tropicalis)* (levures), *Pinus strobus* qui est une espèce d'origine végétale, *Gastropode mollusc, Manduca sexta* qui sont d'origine animale.

De préférence l'enzyme utilisée dans la composition selon l'invention est une alcool oxydase provenant de *Pichia pastoris*.

Généralement, la concentration en enzyme alcool oxydase utilisée dans la composition tinctoriale est comprise entre 0,05% et 20% en poids par rapport au poids total de ladite composition, de préférence comprise entre 0,1 et 10% en poids et de manière encore plus préférée entre 0,5 et 8% en poids par rapport au poids de cette composition.

L'activité enzymatique des enzymes alcool oxydases utilisées conformément à l'invention peut être définie à partir de l'oxydation du donneur en condition aérobie. Unité U correspond à la quantité d'enzyme conduisant à la génération de 1 µmole de peroxyde d'hydrogène par minute à un pH donné et à une température de 25°C.

De façon préférentielle la quantité d'alcool oxydase est comprise entre 10³U et 10⁵ U, de préférence entre 2.10³U et 5.10⁴ U pour 100g de composition tinctoriale.

Le ou les substrats pour ladite enzyme sont encore appelés donneurs pour l'enzyme.

Le substrat pour l'enzyme utilisé dans les compositions selon l'invention est de préférence un alcool choisi parmi les alcools primaires, secondaires, les alcools à longue chaîne hydrocarbonée et les alcools aromatiques. Par exemple à titre de donneur pour les alcool primaire oxydases, on peut citer les alcools primaires contenant de 1 à 6 atomes de carbone ; à titre de donneur pour les aryl alcool oxydases : l'alcool benzylique, le 4-terbutyl benzylique alcool, le 3-hydroxy-4-méthoxybenzyl alcool, le vératryl alcool, le 4-méthoxybenzyl alcool, l'alcool cinnamique ; le 2,4 hexadiéne-1-ol peut également être utilisé en tant que donneur pour les aryl alcool oxydases.

Selon une autre variante de l'invention, le substrat pour l'enzyme est un composé portant au moins une fonction alcool aliphatique ou aromatique, approprié pour réagir avec l'enzyme utilisée. Le composé portant au moins une fonction alcool aliphatique ou aromatique peut notamment être un précurseur de colorant d'oxydation ou encore un adjuvant cosmétiquement acceptable par exemple un polymère, un tensioactif, un conservateur porteur d'au moins une fonction alcool. De façon encore plus préférée, le substrat pour l'enzyme est un précurseur de colorant d'oxydation portant au moins une fonction alcool aliphatique ou aromatique. Par exemple, la N-(β-hydroxypropyl)-paraphénylènediamine qui porte une fonction alcool primaire peut avoir la fonction de base d'oxydation et de substrat pour l'alcool oxydase. De même, les coupleurs d'oxydation, tels que le méta- ou le para-aminophénol, peuvent remplir les deux fonctions. De tels précurseurs sont décrits ci-après. Dans cette variante, l'utilisation d'autres substrats de l'enzyme est facultative.

Ainsi la présente demande vise une composition pour la teinture des fibres kératiniques, en particulier des fibres kératiniques humaines telles que les cheveux, contenant, dans un milieu approprié pour la teinture, au moins les composés suivants : un précurseur de colorant d'oxydation ; au moins une enzyme alcool oxydase ; au moins un substrat, portant une fonction alcool, pour ladite enzyme et au moins un polymère associatif de type polyuréthane, ledit substrat pouvant être substitué (c'est-à-dire remplacé) en tout ou en partie par le précurseur de colorant d'oxydation dans le cas où il porte au moins une fonction alcool aliphatique ou aromatique..

L'utilisation de la composition conforme à l'invention permet de diminuer les risques associés à la manipulation de peroxyde d'hydrogène. De plus, la concentration en conservateurs des compositions selon la présente invention peut être diminuée par l'apport de composés possédant une fonction alcool qui possèdent également des propriétés de conservateur.

Généralement, la concentration du ou des substrats pour l'enzyme est comprise entre 0,01% et 60% en poids par rapport au poids total de la composition et de préférence comprise entre 0,05% et 30% en poids par rapport au poids total de la composition.

Les bases d'oxydation peuvent notamment être choisies parmi les paraphénylènediamines, les bis-phénylalkylènediamines, les para-aminophénols, les ortho-aminophénols et les bases hétérocycliques et leurs sels d'addition.

Parmi les paraphénylènediamines, on peut plus particulièrement citer à titre d'exemple, la paraphénylènediamine, la paratoluylènediamine, la 2-chloro paraphénylènediamine, la 2,3-diméthyl paraphénylènediamine, la 2,6-diméthyl paraphénylènediamine, la 2,6-diéthyl paraphénylènediamine, la 2,5-diméthyl paraphénylènediamine, la N,N-diméthyl paraphénylènediamine, la N,N-diéthyl paraphénylènediamine, la N,N-dipropyl paraphénylènediamine, la 4-amino N,N-diéthyl 3-méthyl aniline, la N,N-bis-(β-hydroxyéthyl) paraphénylènediamine, la 4-N,N-bis-(β-hydroxyéthyl)amino 2-méthyl aniline, la 4-N,N-bis-(β-hydroxyéthyl)amino 2-chloro aniline, la 2-β-hydroxyéthyl paraphénylènediamine, la 2-fluoro paraphénylènediamine, la 2-isopropyl paraphénylènediamine, la N-(β-hydroxypropyl) paraphénylènediamine, la 2-hydroxyméthyl paraphénylènediamine, la N,N-diméthyl 3-méthyl paraphénylènediamine, la N,N-(éthyl, β-hydroxyéthyl) paraphénylènediamine, la N-(β,γ-dihydroxypropyl) paraphénylènediamine, la N-(4'-aminophényl) paraphénylènediamine, la N-phényl paraphénylènediamine, la 2-β-hydroxyéthyloxy paraphénylènediamine, la 2-β-acétylaminoéthyloxy paraphénylènediamine, la N-(β-méthoxyéthyl) paraphénylènediamine, la 4 aminophenyl pyrrolidine, le 2 thiényl paraphénylène diamine, le 2-β hydroxyéthylamino 5-amino toluène, la 3-hydroxy 1-(4' aminophenyl)pyrrolidine et leurs sels d'addition avec un acide.

Parmi les paraphénylènediamines citées ci-dessus, la paraphénylènediamine, la paratoluylènediamine, la 2-isopropyl paraphénylènediamine, la 2-β-hydroxyéthyl paraphénylènediamine, la 2-β-hydroxyéthyloxy paraphénylènediamine, la 2,6-diméthyl paraphénylènediamine, la 2,6-diéthyl paraphénylènediamine, la 2,3-diméthyl paraphénylènediamine, la N,N-bis-(β-hydroxyéthyl) paraphénylènediamine, la 2-chloro paraphénylènediamine, la 2-β-acétylaminoéthyloxy paraphénylènediamine, et leurs sels d'addition avec un acide sont particulièrement préférées.

Parmi les bis-phénylalkylènediamines, on peut citer à titre d'exemple, le N,N'-bis-(β-hydroxyéthyl) N,N'-bis-(4'-aminophényl) 1,3-diamino propanol, la N,N'-bis-(β-hydroxyéthyl) N,N'-bis-(4'-aminophényl) éthylènediamine, la N,N'-bis-(4-aminophényl) tétraméthylènediamine, la N,N'-bis-(β-hydroxyéthyl) N,N'-bis-(4-aminophényl) tétraméthylènediamine, la N,N'-bis-(4-méthyl-aminophényl) tétraméthylènediamine, la N,N'-bis-(éthyl) N,N'-bis-(4'-amino, 3'-méthylphényl) éthylènediamine, le 1,8-bis-(2,5-diamino phénoxy)-3,6-dioxaoctane, et leurs sels d'addition avec un acide.

Parmi les para-aminophénols, on peut citer à titre d'exemple, le para-aminophénol, le 4-amino 3-méthyl phénol, le 4-amino 3-fluoro phénol, le 4-amino 2-chloro phénol, le 4-amino 3-chloro phénol, le 4-amino 3-hydroxyméthyl phénol, le 4-amino 2-méthyl phénol, le 4-amino 2-hydroxyméthyl phénol, le 4-amino 2-méthoxyméthyl phénol, le 4-amino 2-aminométhyl phénol, le 4-amino 2-(β-hydroxyéthyl aminométhyl) phénol, le 4-amino 2-fluoro phénol, le 4 amino 2,6-dichlorophénol, le 4-amino 6[((5'-amino-2'-hydroxy-3'-méthyl)phényl)méthyl]-2-méthylphénol, le bis(5'-amino-2'-hydroxy)phénylméthane et leurs sels d'addition avec un acide.

Parmi les ortho-aminophénols, on peut citer à titre d'exemple, le 2-amino phénol, le 2-amino 5-méthyl phénol, le 2-amino 6-méthyl phénol, le 5-acétamido 2-amino phénol, et leurs sels d'addition avec un acide.

Parmi les bases hétérocycliques, on peut citer à titre d'exemple, les dérivés pyridiniques, les dérivés pyrimidiniques et les dérivés pyrazoliques.

Parmi les dérivés pyridiniques, on peut citer les composés décrits par exemple dans les brevets GB 1 026 978 et GB 1 153 196, comme la 2,5-diamino pyridine, la 2-(4-méthoxyphényl)amino 3-amino pyridine, la 3,4-diamino pyridine, et leurs sels d'addition avec un acide.

D'autres bases d'oxydation pyridiniques utiles dans la présente invention sont les bases d'oxydation 3-amino pyrazolo-[1,5-a]-pyridines ou leurs sels d'addition décrits par exemple dans la demande de brevet FR 2801308. A titre d'exemple, on peut citer la pyrazolo[1,5-a]pyridin-3-ylamine ; la 2-acétylamino pyrazolo-[1,5-a] pyridin-3-ylamine ; la 2-morpholin-4-yl-pyrazolo[1,5-a]pyridin-3-ylamine ; l'acide 3-amino-pyrazolo[1,5-a]pyridin-2-carboxylique ; la 2-méthoxy-pyrazolo[1,5-a]pyridine-3-ylamino ; le (3-amino-pyrazolo[1,5-a]pyridine-7-yl)-méthanol ; le 2-(3-amino-pyrazolo[1,5-a]pyridine-5-yl)-éthanol ; le 2-(3-amino-pyrazolo[1,5-a]pyridine-7-yl)-éthanol ; le (3-amino-pyrazolo[1,5-a]pyridine-2-yl)-méthanol ; la 3,6-diamino-pyrazolo[1,5-a]pyridine ; la 3,4-diamino-pyrazolo[1,5-a]pyridine ; la pyrazolo[1,5-a]pyridine-3,7-diamine ; la 7-morpholin-4-yl-pyrazolo[1,5-a]pyridin-3-ylamine ; la pyrazolo[1,5-a]pyridine-3,5-diamine ; la 5-morpholin-4-yl-pyrazolo[1,5-a]pyridin-3-ylamine ; le 2-[(3-amino-pyrazolo[1,5-a]pyridin-5-yl)-(2-hydroxyéthyl)-amino]-éthanol ; le 2-[(3-amino-pyrazolo[1,5-a]pyridin-7-yl)-(2-hydroxyéthyl)-amino]-éthanol ; la 3-amino-pyrazolo[1,5-a]pyridine-5-ol ; 3-amino-pyrazolo[1,5-a]pyridine-4-ol ; la 3-amino-pyrazolo[1,5-a]pyridine-6-ol ; la 3-amino-pyrazolo[1,5-a]pyridine-7-ol ;
ainsi que leurs sels d'addition avec un acide.

Parmi les dérivés pyrimidiniques, on peut citer les composés décrits par exemple dans les brevets DE 2359399 ; JP 88-169571 ; JP 05-63124 ; EP 0770375 ou demande de brevet WO 96/15765 comme la 2,4,5,6-tétra-aminopyrimidine, la 4-hydroxy 2,5,6-triaminopyrimidine, la 2-hydroxy 4,5,6-triaminopyrimidine, la 2,4-dihydroxy 5,6-diaminopyrimidine, la 2,5,6-triaminopyrimidine et leurs sels d'addition et leurs formes tautomères, lorsqu'il existe un équilibre tautomérique.

Parmi les dérivés pyrazoliques, on peut citer les composés décrits dans les brevets DE 3843892, DE 4133957 et demandes de brevet WO 94/08969, WO 94/08970, FR-A-2 733 749 et DE 195 43 988 comme le 4,5-diamino 1-méthyl pyrazole, le 4,5-diamino 1-(β-hydroxyéthyl) pyrazole, le 3,4-diamino pyrazole, le 4,5-diamino 1-(4'-chlorobenzyl) pyrazole, le 4,5-diamino 1,3-diméthyl pyrazole, le 4,5-diamino 3-méthyl 1-phényl pyrazole, le 4,5-diamino 1-méthyl 3-phényl pyrazole, le 4-amino 1,3-diméthyl 5-hydrazino pyrazole, le 1-benzyl 4,5-diamino 3-méthyl pyrazole, le 4,5-diamino 3-tert-butyl 1-méthyl pyrazole, le 4,5-diamino 1-tert-butyl 3-méthyl pyrazole, le 4,5-diamino 1-(β-hydroxyéthyl) 3-méthyl pyrazole, le 4,5-diamino 1-éthyl 3-méthyl pyrazole, le 4,5-diamino 1-éthyl 3-(4'-méthoxyphényl) pyrazole, le 4,5-diamino 1-éthyl 3-hydroxyméthyl pyrazole, le 4,5-diamino 3-hydroxyméthyl 1-méthyl pyrazole, le 4,5-diamino 3-hydroxyméthyl 1-isopropyl pyrazole, le 4,5-diamino 3-méthyl 1-isopropyl pyrazole, le 4-amino 5-(2'-aminoéthyl)amino 1,3-diméthyl pyrazole, le 3,4,5-triamino pyrazole, le 1-méthyl 3,4,5-triamino pyrazole, le 3,5-diamino 1-méthyl 4-méthylamino pyrazole, le 3,5-diamino 4-(β-hydroxyéthyl)amino 1-méthyl pyrazole, et leurs sels d'addition.

Généralement la concentration de la ou les bases d'oxydation est comprise entre 0,0001 et 20%, de préférence entre 0,005 à 6% en poids par rapport au poids total de la composition.

Parmi les coupleurs d'oxydation, on peut notamment citer les métaphénylènediamines, les méta-aminophénols, les métadiphénols, les coupleurs naphtaléniques et les coupleurs hétérocycliques ainsi que leurs sels d'addition.

A titre d'exemple, on peut citer le 2-méthyl 5-aminophénol, le 5-N-(β-hydroxyéthyl)amino 2-méthyl phénol, le 6-chloro-2-méthyl-5-aminophénol, le 3-amino phénol, le 1,3-dihydroxy benzène (ou résorcinol), le 1,3-dihydroxy 2-méthyl benzène, le 4-chloro 1,3-dihydroxy benzène, le 2,4-diamino 1-(β-hydroxyéthyloxy) benzène, le 2-amino 4-(β-hydroxyéthylamino) 1-méthoxybenzène, le 1,3-diamino benzène, le 1,3-bis-(2,4-diaminophénoxy) propane, la 3-uréido aniline, le 3-uréido 1-diméthylamino benzène, le sésamol, le 1-β-hydroxyéthylamino-3,4-méthylènedioxybenzène, l'α-naphtol, le 2 méthyl-1-naphtol, le 6-hydroxy indole, le 4-hydroxy indole, le 4-hydroxy N-méthyl indole, la 2-amino-3-hydroxy pyridine, la 6-hydroxy benzomorpholine, la 3,5-diamino-2,6-diméthoxypyridine, le 1-N-(β-hydroxyéthyl)amino-3,4-méthylène dioxybenzène, le 2,6-bis-(β-hydroxyéthylamino)toluène et leurs sels d'addition.

Généralement la concentration du ou des coupleurs d'oxydation est comprise entre 0,0001 et 20%, de préférence entre 0,005 à 6% en poids par rapport au poids total de la composition.

D'une manière générale, les sels d'addition avec un acide utilisables pour les bases d'oxydation et les coupleurs sont notamment choisis parmi les chlorhydrates, les bromhydrates, les sulfates, les citrates, les succinates, les tartrates, les lactates, les tosylates, les benzènesulfonates, les phosphates et les acétates.

Les sels d'addition avec une base utilisables dans le cadre de l'invention sont par exemple choisis parmi les sels d'addition avec la soude, la potasse, l'ammoniaque, les amines ou les alcanolamines.

La composition tinctoriale conforme à l'invention peut en outre contenir un ou plusieurs colorants directs pouvant notamment être choisis parmi les colorants nitrés de la série benzénique, neutres, acides ou cationiques, les colorants directs azoïques neutres, acides ou cationiques, les colorants directs quinoniques et en particulier anthraquinoniques neutres, acides ou cationiques, les colorants directs aziniques, les colorants directs méthiniques, azométhiniques, triarylméthaniques, indoaminiques et les colorants directs naturels. De préférence, ces colorants directs additionnels sont choisis parmi les colorants directs cationiques et les colorants directs naturels.

Parmi les colorants directs cationiques utilisables selon l'invention on peut citer les colorants directs azoïques cationiques décrits dans les demandes de brevets WO 95/15144, WO-95/01772 et EP-714954.

Parmi ces composés on peut tout particulièrement citer les colorants suivants :
- chlorure de 1,3-diméthyl-2-[[4-(diméthylamino)phényl]azo]-1H-Imidazolium,
- chlorure de 1,3-diméthyl-2-[(4-aminophényl)azo]-1H-Imidazolium,
- méthylsulfate de 1-méthyl-4-[(méthylphénylhydrazono) méthyl]-pyridinium.

Parmi les colorants directs naturels utilisables selon l'invention, on peut citer la lawsone, la juglone, l'alizarine, la purpurine, l'acide carminique, l'acide kermésique, la purpurogalline, le protocatéchaldéhyde, l'indigo, l'isatine, la curcumine, la spinulosine, l'apigénidine. On peut également utiliser les extraits ou décoctions contenant ces colorants naturels et notamment les cataplasmes ou extraits à base de henné.

Le ou les colorants directs représentent de préférence de 0,001 à 20% en poids environ du poids total de la composition prête à l'emploi et encore plus préférentiellement de 0,005 à 10% en poids environ.

La composition tinctoriale conforme à l'invention peut également renfermer divers adjuvants additionnels, différents des polymères associatifs de type polyuréthanes. Ces agents additionnels sont utilisés classiquement dans les compositions pour la teinture des cheveux, ce sont par exemple des agents antioxydants, des agents de pénétration, des agents séquestrants, des parfums, des tampons, des agents dispersants, des tensioactifs, des agents conditionneurs tels que par exemple des silicones volatiles ou non volatiles, modifiées ou non modifiées, des polymères cationiques, des cations, des agents filmogènes, des céramides, des agents conservateurs, des agents opacifiants, des vitamines ou provitamines.

Les adjuvants ci-dessus sont en général présents en quantité comprise pour chacun d'eux entre 0,01 et 20 % en poids par rapport au poids de la composition.

Bien entendu, l'homme de l'art veillera à choisir ce ou ces éventuels composés complémentaires de manière telle que les propriétés avantageuses attachées intrinsèquement à la composition de teinture d'oxydation conforme à l'invention ne soient pas, ou substantiellement pas, altérées par la ou les adjonctions envisagées.

Le milieu approprié pour la teinture appelé aussi support de teinture est généralement constitué par de l'eau ou par un mélange d'eau et d'au moins un solvant organique pour solubiliser les composés qui ne seraient pas suffisamment solubles dans l'eau. Ce solvant peut être le cas échéant un substrat de l'enzyme comme l'éthanol, l'isopropanol. Cela peut être également un composé non substrat de l'enzyme tel que les éthers de polyols comme le 2-butoxyéthanol, le propylèneglycol, le monométhyléther de propylèneglycol, le monoéthyléther et le monométhyléther du diéthylèneglycol, le phénoxyéthanol, et leurs mélanges.

Les solvants peuvent être présents dans des proportions de préférence comprises entre 1 et 40 % en poids environ par rapport au poids total de la composition tinctoriale, et encore plus préférentiellement entre 5 et 30 % en poids environ.

Le pH de la composition tinctoriale conforme à l'invention est généralement compris entre 6 et 11 environ, et de préférence entre 7 et 10 environ. Il peut être ajusté à la valeur désirée au moyen d'agents acidifiants ou alcalinisants habituellement utilisés en teinture des fibres kératiniques ou bien encore à l'aide de systèmes tampons classiques.

Parmi les agents acidifiants, on peut citer, à titre d'exemple, les acides minéraux ou organiques comme l'acide chlorhydrique, l'acide orthophosphorique, l'acide sulfurique, les acides carboxyliques comme l'acide acétique, l'acide tartrique, l'acide citrique, l'acide lactique, les acides sulfoniques.

Parmi les agents alcalinisants on peut citer, à titre d'exemple, l'ammoniaque, les carbonates alcalins, les alcanolamines telles que les mono-, di- et triéthanolamines ainsi que leurs dérivés, les hydroxydes de sodium ou de potassium et les composés de formule (III) suivante : 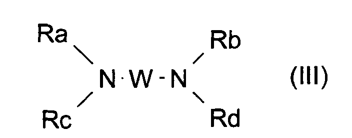 dans laquelle W est un reste propylène éventuellement substitué par un groupement hydroxyle ou un radical alkyle en C₁-C₄ ; Ra, Rb, Rc et Rd, identiques ou différents, représentent un atome d'hydrogène, un radical alkyle en C₁-C₄ ou hydroxyalkyle en C₁-C₄.

La composition tinctoriale peut se présenter sous des formes diverses, telles que sous forme de liquides épaissis, de crèmes, de gels, ou sous toute autre forme appropriée pour réaliser une teinture des fibres kératiniques, et notamment des cheveux humains.

Lorsque les colorants d'oxydation et la ou les alcool oxydases sont présents au sein de la même composition prête à l'emploi, ladite composition est de préférence exempte d'oxygène gazeux de manière à éviter toute oxydation prématurée du ou des colorants d'oxydation.

L'invention a également pour objet un procédé de teinture des fibres kératiniques, et en particulier des fibres kératiniques humaines telles que les cheveux, tel qu'on applique sur ces fibres au moins une composition pour la teinture selon l'invention, la durée de cette application étant la durée suffisante pour développer la coloration désirée.

La couleur est alors révélée par la mise en présence de l'enzyme alcool oxydase et de son substrat en présence d'oxygène.

La composition est appliquée sur les fibres kératiniques. Après un temps de pose de 3 à 60 minutes environ, de préférence 5 à 40 minutes environ, les fibres kératiniques sont rincées, lavées au shampooing, rincées à nouveau puis séchées.

Lorsque la composition pour la teinture est une composition sous une forme prête à l'emploi, elle comprend, dans un milieu approprié pour la teinture des fibres kératiniques, au moins un précurseur de colorant d'oxydation, au moins une enzyme alcool oxydase, au moins un substrat pour ladite enzyme et au moins un polymère associatif de type polyuréthane, l'ensemble est alors stocké sous forme anaérobie, exempte d'oxygène gazeux.

Selon une variante, ce procédé comporte une étape préliminaire consistant à stocker sous forme séparée, d'une part une composition (A) comprenant, dans un milieu approprié pour la teinture des fibres kératiniques, au moins un précurseur de colorant d'oxydation et d'autre part, une composition (B) renfermant, dans un milieu approprié pour la teinture des fibres kératiniques, au moins une enzyme alcool oxydase, la composition (A) et/ou la composition (B) contenant au moins un substrat pour ladite enzyme, et la composition (A) et/ou la composition (B) contenant au moins un polymère associatif de type polyuréthane ; puis à procéder au mélange des compositions (A) et (B) au moment de l'emploi avant d'appliquer ce mélange sur les fibres kératiniques.

Selon une variante de l'invention, le procédé comporte une étape préliminaire consistant à stocker sous forme séparée, d'une part une composition (A) comprenant, dans un milieu approprié pour la teinture des fibres kératiniques, au moins un précurseur de colorant d'oxydation, au moins un substrat pour ladite enzyme et au moins un polymère associatif de type polyuréthane et d'autre part, une composition (B) renfermant, dans un milieu approprié pour la teinture des fibres kératiniques, au moins une enzyme alcool oxydase ; puis à procéder au mélange des compositions (A) et (B) au moment de l'emploi avant d'appliquer ce mélange sur les fibres kératiniques.

La couleur peut être révélée à pH acide, neutre ou alcalin. Dans le cas où le procédé est mis en oeuvre au moyen d'une composition (A) comprenant au moins un précurseur de colorant d'oxydation, au moins un substrat pour ladite enzyme et au moins un polymère associatif de type polyuréthane et d'une composition (B) renfermant au moins une enzyme alcool oxydase, l'enzyme peut être ajoutée à la composition de l'invention juste au moment de l'emploi ou peut être mise en oeuvre à partir d'une composition la contenant, appliquée simultanément ou séquentiellement à la composition de l'invention.

Cette composition B (dite composition oxydante) peut également renfermer divers adjuvants utilisés classiquement dans les compositions pour la teinture des cheveux et tels que définis précédemment.

Le pH de la composition B dite oxydante est tel qu'après mélange avec la composition tinctoriale A, le pH de la composition résultante appliquée sur les fibres kératiniques varie de préférence entre 6 et 11 environ, et encore plus préférentiellement entre 7 et 10. Il peut être ajusté à la valeur désirée au moyen d'agents acidifiants ou alcalinisants habituellement utilisés en teinture des fibres kératiniques et tels que définis précédemment.

De préférence, l'application de la composition selon l'invention est effectuée à une température comprise entre la température ambiante et 220°C, de préférence entre la température ambiante et 60°C.

Un autre objet de l'invention est un dispositif à plusieurs compartiments ou "kit" de teinture dans lequel un premier compartiment renferme la composition (A) telle que définie ci-dessus et un deuxième compartiment renferme la composition (B) telle que définie ci-dessus. Ce dispositif peut être équipé d'un moyen permettant de délivrer sur les cheveux le mélange souhaité, tel que les dispositifs décrits dans le brevet FR-2 586 913 au nom de la demanderesse.

Les exemples qui suivent servent à illustrer l'invention sans toutefois présenter un caractère limitatif.

### EXEMPLES

On prépare la composition suivante

| Constituants | Composition 1 |
|---|---|
| VISCOPHOBE® DB 1000 (Union Carbide) | 1 % |
| Ethanol | 25g |
| paraphénylènediamine | 3.10⁻³ mole |
| métaaminophénol | 3.10⁻³ mole |
| Alcool oxydase | 20000unités |
| 2-amino-2-méthyl-1-propanol | qs pH 7 |
| Eau distillée | qsp 100g |

L'alcool oxydase utilisée est celle commercialisée par la société Biozyme Laboratories sous forme liquide à la concentration de 1980 unités/ml.

L'unité U correspond à la quantité d'enzyme conduisant à la génération de 1 µmole de peroxyde d'hydrogène par minute à pH 7,5 (tampon phosphate 100mM) et à une température de 25°C.

On applique les compositions ci-dessus sur des mèches de cheveux gris naturels et permanentés à 90% de blancs et on laisse poser pendant 30 minutes. Le rapport de bain est fixé à 5. L'alcool oxydase est ajoutée extemporanément. Les cheveux ont ensuite été rincés, lavés avec un shampooing standard, puis séchés.

Les cheveux ont été teints dans des nuances vert kaki.

## Revendications

1. Composition pour la teinture des fibres kératiniques, en particulier des fibres kératiniques humaines telles que les cheveux, contenant, dans un milieu approprié pour la teinture, au moins les composés suivants : un précurseur de colorant d'oxydation ; au moins une enzyme alcool oxydase ; au moins un substrat, portant une fonction alcool, pour ladite enzyme et au moins un polymère associatif de type polyuréthane, ledit substrat pouvant être substitué en tout ou en partie par le précurseur de colorant d'oxydation dans le cas où ledit précurseur porte au moins une fonction alcool aliphatique ou aromatique.

2. Composition selon la revendication 1 telle que le polymère associatif de type polyuréthane est un polyuréthane cationique de formule (I)
R-X-(P)n-[L-(Y)m]r-L'-(P')p-X'-R' (I)
dans laquelle :
R et R', identiques ou différents, représentent un groupement hydrophobe ou un atome d'hydrogène ;
X et X', identiques ou différents, représentent un groupement comportant une fonction amine portant ou non un groupement hydrophobe, ou encore le groupement L" ;
L, L' et L", identiques ou différents, représentent un groupement dérivé d'un diisocyanate ;
P et P', identiques ou différents, représentent un groupement comportant une fonction amine portant ou non un groupement hydrophobe ;
Y représente un groupement hydrophile ;
r est un nombre entier compris entre 1 et 100, de préférence entre 1 et 50 et en particulier entre 1 et 25,
n, m, et p valent chacun indépendamment des autres entre 0 et 1000 ; la molécule contenant au moins une fonction amine quaternaire ou quaternisée et au moins un groupement hydrophobe.

3. Composition selon la revendication 2 telle que les seuls groupements hydrophobes sont les groupes R et R' aux extrémités de la chaîne.

4. Composition selon la revendication 2 ou 3 telle que R et R' représentent tous les deux indépendamment un groupement hydrophobe, X, X' représentent chacun un groupe L", n et p valent entre 1 et 1000 et L, L', L", P, P', Y et m ont la signification indiquée à la revendication 2.

5. Composition selon la revendication 2 ou 3 telle que R et R' représentent tous les deux indépendamment un groupement hydrophobe, X, X' représentent chacun un groupe L", n et p valent 0, et L, L', L", Y et m ont la signification indiquée à la revendication 2.

6. Composition selon la revendication 2 ou 3 telle que R et R' représentent tous les deux indépendamment un groupement hydrophobe, X et X' représentent tous les deux indépendamment un groupement comportant une amine quaternaire, n et p valent 0, et L, L', Y et m ont la signification indiquée à la revendication 2.

7. Composition selon l'une des revendications 2 à 6 telle qu'ils présentent une masse moléculaire moyenne en nombre comprise entre 400 et 500 000, de préférence entre 1000 et 400 000 et en particulier entre 1000 et 300 000.

8. Composition selon l'une des revendications 2 à 7 telle que R et R' représentent un radical ou polymère à chaîne hydrocarbonée, saturée ou non, linéaire ou ramifiée, pouvant contenir un ou plusieurs hétéroatomes tels que P, O, N, S, ou un radical à chaîne perfluorée ou siliconée.

9. Composition selon l'une des revendications 2 à 8 telle que X et/ou X' représentent l'une des formules suivantes : 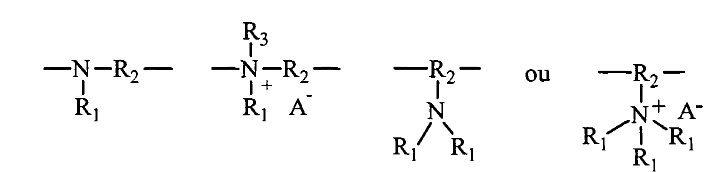 pour X 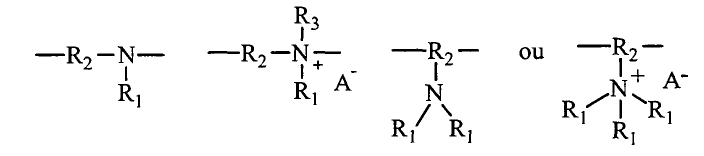 pour X'
dans lesquelles :
R₂ représente un radical alkylène ayant de 1 à 20 atomes de carbone, linéaire ou ramifié, comportant ou non un cycle saturé ou insaturé, ou un radical arylène, un ou plusieurs des atomes de carbone pouvant être remplacé par un hétéroatome choisi parmi N, S, O, P ;
R₁ et R₃, identiques ou différents, désignent un radical alkyle ou alcényle en C₁-C₃₀, linéaire ou ramifié, un radical aryle, pouvant contenir un ou plusieurs hétéroatomes choisis parmi N, S, O, P ; A⁻ est un contre-ion physiologiquement acceptable.

10. Composition selon l'une des revendications 2 à 8 telle que les groupements L, L' et L" représentent un groupe de formule : 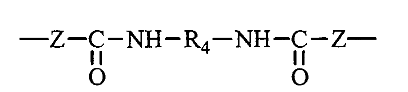 dans laquelle :
Z représente ―O-, -S- ou ―NH- ; et R₄ représente un radical alkylène ayant de 1 à 20 atomes de carbone, linéaire ou ramifié, comportant ou non un cycle saturé ou insaturé, ou un radical arylène, pouvant contenir un ou plusieurs hétéroatomes (1,2,3).

11. Composition selon l'une des revendications 2 à 10 telle que les groupements P et P', identiques ou différents, représentent au moins l'une des formules suivantes : 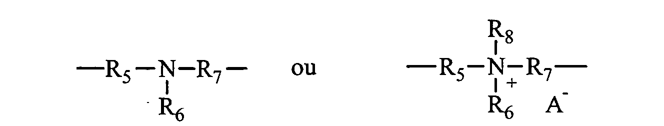 ou 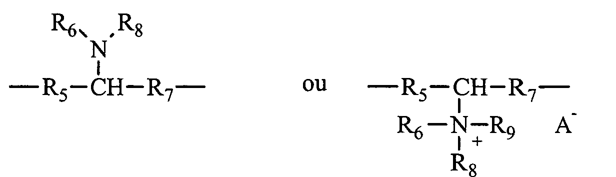 ou 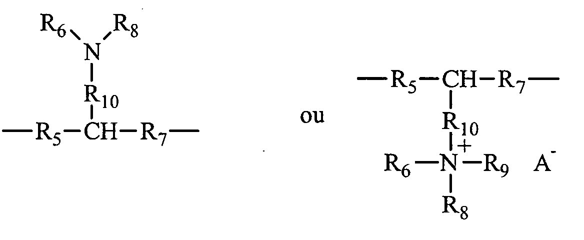 dans lesquelles :
R₅ et R₇ ont les mêmes significations que R₂ défini à la revendication 9 ; R₆, R₈ et R₉ ont les mêmes significations que R₁ et R₃ définis à la revendication 9 ; R₁₀ représente un groupe alkylène, linéaire ou ramifié, éventuellement insaturé et pouvant contenir un ou plusieurs hétéroatomes choisis parmi N, O, S et P, et A⁻ est un contre-ion physiologiquement acceptable.

12. Composition selon l'une des revendications 2 à 11 telle que Y représente un groupe dérivé d'éthylèneglycol, de diéthylèneglycol ou de propylèneglycol, ou un groupe dérivé d'un polymère choisi parmi les polyéthers, les polyesters sulfonés et les polyamides sulfonés.

13. Composition selon la revendication 1 telle que le polymère associatif est un polyuréthane anionique de type terpolymère acrylique comprenant :
a) environ 20 à 70% en poids d'un acide carboxylique à insaturation α, β-monoéthylénique ;
b) environ 20 à 80% en poids d'un monomère à insaturation monoéthylénique non tensioactif différent de a) et
c) environ 0,5 à 60% en poids d'un monomère uréthane non-ionique qui est le produit de réaction d'un tensioactif non-ionique monohydrique avec un monoisocyanate à insaturation monoéthylénique.

14. Composition selon la revendication 13, **caractérisée en ce que** le polyuréthane associatif anionique est un copolymère acide méthacrylique/méthacrylate de méthyle/méthylstyrène-isopropyl-isocyanate/alcool béhénylique polyéthoxylé comportant 40 motifs éthoxy.

15. Composition selon la revendication 1, **caractérisée en ce que** le polymère associatif est un polyuréthane non ionique de type polyuréthane-polyéther susceptible d'être obtenu par polycondensation d'au moins trois composés comprenant (i) au moins un polyéthylèneglycol comprenant de 150 à 180 moles d'oxyde d'éthylène, (ii) de l'alcool stéarylique ou de l'alcool décylique et (iii) au moins un diisocyanate.

16. Composition selon la revendication 15, **caractérisée en ce que** le polyuréthane associatif non ionique est un polycondensat de polyéthylèneglycol à 150 ou 180 moles d'oxyde d'éthylène, d'alcool stéarylique et de méthylène-bis(4-cyclohexylisocyanate) ou un polycondensat de polyéthylèneglycol à 150 ou 180 moles d'oxyde d'éthylène, d'alcool décylique et de méthylène-bis(4-cyclohexylisocyanate).

17. Composition selon l'une quelconque des revendications précédentes telle que le polymère associatif de type polyuréthane est présent en une quantité comprise entre 0,01 et 10%, de préférence entre 0,1 et 5% en poids par rapport au poids total de la composition de teinture.

18. Composition selon l'une quelconque des revendications précédentes telle que l'enzyme alcool oxydase est choisie parmi les alcool primaire oxydases (EC1.1.3.13), les alcool secondaire oxydases (EC 1.1.3.18), les alcool à longue chaîne hydrocarbonée oxydases (EC 1.1.3.20), les alcool polyvinylique oxydases (EC 1.1.3.30), l'alcool vanillique oxydase (EC 1.1.3.38), les alcool aromatique oxydases (EC 1.1.3.7).

19. Composition selon la revendication 18 telle l'enzyme alcool oxydase est issue d'une des espèces suivantes : *Rhodococcus erythropolis, Pseudomonas pseudoalcaligenes, Aspergillus niger, Kamagataella pastoris, Phanerochaete chrysosporium, Polyporus obtusus, Hansenula polymorpha, Poria contigua, Penicillium simplicissimum, Pleurotus pulmonarius, Pichia sp. (pastoris, methanolica, angusta)* et *Candida sp. (boidinii, albicans, tropicalis) , Pinus strobus, Gastropode mollusc, Manduca sexta* ,de préférence *Pichia pastoris*.

20. Composition selon l'une des revendications 18 ou 19, telle que la concentration en enzyme alcool oxydase est comprise entre 0,05 et 20%, de préférence entre 0,1 et 10% et de manière encore plus préférée entre 0,5 et 8% en poids par rapport au poids total de ladite composition.

21. Composition selon l'une des revendications précédentes telle que la quantité d'alcool oxydase est comprise entre 10³U et 10⁵U, de préférence entre 2.10³U et 5.10⁴U pour 100g de composition tinctoriale.

22. Composition selon l'une quelconque des revendications précédentes telle que le substrat pour l'enzyme est un alcool choisi parmi les alcools primaires, secondaires, les alcools à longue chaîne hydrocarbonée et les alcools aromatiques.

23. Composition selon la revendication 22 telle que la concentration du ou des substrats pour l'enzyme est comprise entre 0,01% et 60% en poids par rapport au poids total de la composition et de préférence comprise entre 0,05% et 30% en poids par rapport au poids total de la composition.

24. Composition selon l'une quelconque des revendications précédentes telle qu'elle comprend en tant que précurseur de colorant d'oxydation une base d'oxydation choisie parmi les paraphénylènediamines, les bis-phénylalkylènediamines, les para-aminophénols, les ortho-aminophénols, les bases hétérocycliques et leurs sels d'addition.

25. Composition selon la revendication 24 telle que la concentration de cette base d'oxydation est comprise entre 0,0001 et 20% en poids par rapport au poids total de la composition.

26. Composition selon l'une quelconque des revendications précédentes telle que la composition comprend en tant que précurseur de colorant d'oxydation un coupleur d'oxydation classique choisi parmi les métaphénylènediamines, les métaaminophénols, les métadiphénols, les coupleurs naphtaléniques, les coupleurs hétérocycliques ainsi que leurs sels d'addition.

27. Composition selon la revendication 26 telle que la concentration du ou des coupleurs est comprise entre 0,0001 et 20 % en poids par rapport au poids total de la composition.

28. Composition selon l'une des revendications précédentes **caractérisée par le fait qu'**elle renferme un ou plusieurs colorants directs naturels ou cationiques.

29. Procédé de teinture des fibres kératiniques et en particulier des fibres kératiniques humaines telles que les cheveux, **caractérisée par le fait qu'**on applique sur lesdites fibres au moins une composition selon l'une des revendications 1 à 28, pendant une durée suffisante pour développer la coloration désirée.

30. Procédé selon la revendication 29, **caractérisé par le fait que** la composition est une composition prête à l'emploi comprenant une composition selon l'une des revendications 1 à 28, l'ensemble étant stocké sous forme anaérobie, exempte d'oxygène gazeux.

31. Procédé selon la revendication 32, **caractérisée par le fait qu'**il comporte une étape préliminaire consistant à stocker sous forme séparée, d'une part une composition (A) comprenant, dans un milieu approprié pour la teinture, au moins un précurseur de colorant d'oxydation et d'autre part, une composition (B) renfermant, dans un milieu approprié pour les fibres kératiniques, au moins une enzyme alcool oxydase, la composition (A) et/ou la composition (B) contenant au moins un substrat pour ladite enzyme et la composition (A) et/ou la composition (B) contenant au moins un polymère associatif de type polyuréthane, puis à procéder au mélange des compositions (A) et (B) au moment de l'emploi avant d'appliquer ce mélange sur les fibres kératiniques.

32. Procédé selon la revendication 31, **caractérisée par le fait qu'**il comporte une étape préliminaire consistant à stocker sous forme séparée, d'une part une composition (A) comprenant, dans un milieu approprié pour la teinture, au moins un précurseur de colorant d'oxydation, au moins un substrat pour ladite enzyme et au moins un polymère associatif de type polyuréthane et d'autre part, une composition (B) renfermant, dans un milieu approprié pour les fibres kératiniques, au moins une enzyme alcool oxydase, puis à procéder au mélange des compositions (A) et (B) au moment de l'emploi avant d'appliquer ce mélange sur les fibres kératiniques.

33. Dispositif à plusieurs compartiments ou « Kit » de teinture, **caractérisé par le fait qu'**il comporte un premier compartiment renfermant la composition (A) telle que définie à l'une des revendications 31 ou 32 et un second compartiment renfermant la composition (B) telle que définie à l'une des revendications 31 ou 32.
